# EUROPEAN PATENT SPECIFICATION

(11) **EP 2 171 086 B1**
(45) Date of publication and mention of the grant of the patent: **19.12.2012**
(21) Application number: 08771019.0
(22) Date of filing: 13.06.2008
(51) Int. Cl.: C12Q 1/68

(54) **METHODS OF DIAGNOSING AND TREATING CANCER**
VERFAHREN ZUR DIAGNOSE UND BEHANDLUNG VON KREBS
PROCÉDÉS DE DIAGNOSTIC ET TRAITEMENT DU CANCER

(30) Priority: 15.06.2007 US 944157 P; 26.03.2008 US 39555
(43) Date of publication of application: 07.04.2010
(73) Proprietor: University of South Florida, Tampa, FL 33612-9220 (US)
(72) Inventor: BEPLER, Gerold, Detroit, MI 48201 (US)
(74) Representative: Williams, Gareth Owen
(86) International application number: PCT/US2008/066918
(87) International publication number: WO 2008/157353

(56) References cited:
- US-A1- 2002 090 642
- US-A1- 2006 252 082
- GIOVANNETTI ELISA ET AL: "Cellular and pharmacogenetics foundation of synergistic interaction of pemetrexed and gemcitabine in human non-small-cell lung cancer cells" MOLECULAR PHARMACOLOGY, vol. 68, no. 1, July 2005 (2005-07), pages 110-118 URL, XP002513966 ISSN: 0026-895X
- MARING J G ET AL: "Genetic factors influencing pyrimidine-antagonist chemotherapy" PHARMACOGENOMICS JOURNAL, vol. 5, no. 4, 2005, pages 226-243, XP008126509 ISSN: 1470-269X
- GUSTAVSON ET AL.: 'AQUATM based analysis of thymidylate synthase (TS) within subcellular compartments reveals a novel biomarker for prediction of survival in colorectal carcinomas' FIRST AACR INTERNATIONAL CONFERENCE ON MOLECULAR DIAGNOSTICS IN CANCER THERAPEUTIC DEVELOPMENT, AACR MEETING ABSTRACTS 2006, [Online] 12 September 2006 - 15 September 2006, XP008126759 Retrieved from the Internet: <URL:http://www.aacrmeetingabstracts.org/cg i/content/abstract/2006/2/A35>
- ROSELL RAFAEL ET AL: "The promise of pharmacogenomics: gemcitabine and pemetrexed", ONCOLOGY, PRR, HUNTINGTON, NY, US, vol. 18, no. 13 Suppl 8, 4 November 2004 (2004-11-04), pages 70-76, XP008098353, ISSN: 0890-9091
- BEPLER GEROLD ET AL: "Clinical efficacy and predictive molecular markers of neoadjuvant gemcitabine and pemetrexed in resectable non-small cell lung cancer.", JOURNAL OF THORACIC ONCOLOGY : OFFICIAL PUBLICATION OF THE INTERNATIONAL ASSOCIATION FOR THE STUDY OF LUNG CANCER OCT 2008 LNKD- PUBMED:18827606, vol. 3, no. 10, October 2008 (2008-10), pages 1112-1118, ISSN: 1556-1380
- LORD R V N ET AL: "LOW ERCC1 EXPRESSION CORRELATES WITH PROLONGED SURVIVAL AFTER CISPLATIN PLUS GEMCITABINE CHEMOTHERAPY IN NON-SMALL CELL LUNG CANCER", CLINICAL CANCER RESEARCH, THE AMERICAN ASSOCIATION FOR CANCER RESEARCH, US, vol. 8, no. 7, 1 July 2002 (2002-07-01), pages 2286-2291, XP001120598, ISSN: 1078-0432

## Description

### CLAIM OF PRIORITY

This application claims the benefit of U.S. Provisional Patent Application Serial Nos. 60/944,157, filed on June 15, 2007, and 61/039,555, filed on March 26, 2008.

### FEDERALLY SPONSORED RESEARCH OR DEVELOPMENT

This invention was made With Government support under Grant Nos. RO1 CA102726 awarded by the National Cancer Institute of the National Institutes of Health. The Government has certain rights in the invention.

### TECHNICAL FIELD

This invention relates to methods of diagnosing and treating cancer, e.g., non-small cell lung carcinoma.

### BACKGROUND

Lung cancer is the most common cause of cancer death in the United States. Non-small cell lung cancer (NSCLC) is the most common type of lung cancer, accounting for over 80% of lung cancers. It generally grows and spreads more slowly than small cell lung cancer (SCLC). There are three major forms of NSCLC, including adenocarcinomas, often found in an outer area ot the lung; squamous cell carcinomas, usually found in the center of the lung by a bronchus; and large cell carcinomas, which can occur in any part of the lung and tend to grow and spread faster than the other two types of NSCLC. See, e.g., Travis et al., Cancer 75(Suppl. 1):191-202 (1995).

Recent work has demonstrated that genes involved in nucleotide metabolism and DNA repair are important determinants of the phenotypic behavior of early-stage non-small-cell lung cancer (NSCLC). Specifically, Ribonucleotide Reductase M1 (RRM1), the regulatory subunit of ribonucleotide reductase, and ERCCI, a component of the 5' nuclease involved in nucleotide excision repair, are prognostic of patients outcomes (Bepler et al., J Clin Oncol;22:1878-1885 (2004); Simon et al., Chest 127(3):978-83 (2005); Olaussen et al., N Engl J Med 355:983-991 (2006); Zheng et al., N Engl J Med. 356:800-808 (2007)). High levels of mRNA and protein expression of these genes are associated with long survival in patients and reduced metastasis formation in animal models (Gautam et al., Oncogene 22:2135-2142 (2063)). In addition, high RRM1 levels in transgenic animals were found to be protective of carcinogen-induced lung tumor formation (Gautam et al., Cancer Res 66:6497-6502 (2006)).

Giovannetti et al, Molecular Pharmacology volume 68, No: 1, July 2005 pages 110 to 118 discloses that sensitivity to gemcitabine correlates with expression of RRM1 in cell culture, and that a similar correlation is found between pemetrexed and TS expression. The publication suggests that these findings may have implications for rational design of future drug regimens incorporating gemcitabine and pemetrexed.

Rosell et al, Oncology, volume 18, No: 13 supplement 8, November 2004 pages 17 to 76 indicates that the assessment of RRM1 and TS mRNA expression levels might select patients who benefit from gemcitabine or pemetrexed combinations.

### SUMMARY OF THE INVENTION

According to the present invention there is provided a method for predicting a subject's response to a treatment comprising administration of gencetamine and pemetrexed, the method being as set out in independent claim 1. Further features of the invention are disclosed in the dependent claims.

The present invention is based on the discovery that expression levels of thymidylate synthase (TS) are prognostic of outcome in subjects with NSCLC, e.g., early stage (stage I) NSCLC. In addition, the level of expression of TS and RRM1 is correlated with response to pre-operative treatment with a non-platinum doublet, namely gemcitabine and pemetrexed, in patients with surgically resectable NSCLC.

Thus, described herein are methods of predicting outcome in subjects with NSCLC, e.g., early stage (stage I) NSCLC.

Also disclosed are methods of selecting patients for perioperative treatment, e.g., pre- or post-operative treatment, with non-platinum antimetabolite agent, e.g., one or both of gemcitabine and pernetrexed. The method includes determining levels of intratumoral expression of TS, or TS and RRM1, and selecting a patient based on the expression levels.

In another aspect, the disclosure provides methods for selecting an appropriate chemotherapy for a subject. The methods include obtaining a tumor sample from the subject; determining a level of Ribonucleotide Reductase M1 (RRM1) and thymidylate synthase (TS) gene expression in the tumor sample; and selecting an appropriate chemotherapy based on the RRM1 and TS expression levels. If the RRM1 and TS expression levels are less than or equal to the median RRM1 expression level of a reference cohort, then a chemotherapy comprising gemcitabine and pemetrexed is selected. In some embodiments, the methods further include administering the selected appropriate chemotherapy to the subject. In some embodiments, the methods can also include administering a second chemotherapeutic agent to the subject, e.g., an antitubulin or platinum-containing agent.

In an additional aspect, the disclosure provides methods of treating a subject with cancer, e.g., NSCLC, e.g., operable NSCLC. The method includes selecting a subject based on the presence of low expression levels of TS and RRM1, and administering to the subject a combination of gemcitabine and pemetrexed, e.g., as four bi-weekly treatments. In some embodiments, the methods further include determining levels of intratumoral expression of TS, or TS and RRM1.

In a further aspect, the disclosure provides methods of predicting a subject's response to a treatment, e.g., a pre- or post-operative treatment, with a non-platinum antimetabolite agent, e.g., one or both of gemcitabine and pemetrexed. In a further aspect, the disclosure provides methods for predicting a subject's response to a treatment comprising administration of gemcitabine and pemetrexed. The methods include obtaining a tumor sample from the subject; determining a level of Ribonucleotide Reductase M1 (RRM1) and thymidylate synthase (TS) gene expression in the tumor sample; and predicting the subject's response to the treatment based on the level of RRM1 and TS gene expression in the tumor sample. Low levels of expression of RRM1 and TS indicate that the subject is likely to have a positive response to the treatment.

In yet another aspect, the disclosure provides methods for providing a prognosis for a subject diagnosed with non-small cell lung cancer. The methods include obtaining a tumor sample from the subject; determining a level of cytoplasmic thymidylate synthase (TS) protein in the tumor sample; and comparing the level of cytoplasmic TS protein in the sample to a reference level of cytoplasmic TS protein. A high level of cytoplasmic TS protein in the sample as compared to the reference level is indicative of a good prognosis, and a low level of cytoplasmic TS protein as compared to the reference level is indicative of a poor prognosis. In some embodiments, the level of cytoplasmic TS protein is determined using a quantitative in situ analysis method, e.g., AQUA as described herein.

In another aspect, the disclosure provides methods of determining or monitoring the effectiveness of a treatment, e.g., a pre- or post-operative treatment, with a non-platinum antimetabolite agent, e.g., one or both of gemcitabine and pernetrexed. The methods include determining a level of TS and or RRM1, prior to the treatment to establish a baseline level, and determining one or more levels of TS and/or RRM1, after the treatment is initiated. A change in the levels of the measured gene is indicative of the efficacy of the treatment; for example, an increase in expression of TS and/or RRM1 indicates that the treatment is effective. The method can also include making a treatment decision based on changes in levels of TS and/or RRM1.

In another aspect, the disclosure provides kits including a reagent for assaying RRM1 and TS expression in a tissue sample from a patient, and an instruction sheet. In some embodiments, the reagents for assaying RRM1 and TS expression comprise premeasured portions of reagent selected from the group selected from oligo-dT primers, forward primers that hybridize to RRM1 or TS cDNAs, reverse that hybridize to RRM1 or TS cDNAs, reverse transcriptases, DNA polymerases, buffers, and nucleotides. In some embodiments, the reagents for assaying RRM1 and TS expression comprise premeasured portions of anti-RRM1 and anti-TS antibodies and buffers for performing a Western blot or immunohistochemistry assay. In some embodiments, the kits also include a reagent for processing a tissue sample from a patient.

A "proliferative disorder," is a disorder characterised by irregularities in cell division. A cancer (*e.g*., a glioma, prostate cancer, melanoma, carcinoma, cervical cancer, breast cancer, colon cancer, or sarcoma) is an example of a proliferative disorder. Cells characteristic of proliferative disorders (*i.e.*, "neoplastic cells" or "tumor cells") have the capacity for autonomous growth, *i.e.*, an abnormal state or condition characterized by inappropriate proliferative growth of cell populations. A neoplastic cell or a tumor cell is a cell that proliferates at an abnormally high rate, A new growth comprising neoplastic cells is a neoplasm, also known as a "tumor." A tumor is an abnormal tissue growth, generally forming a distinct mass, that grows by cellular proliferation more rapidly than normal tissue. A tumor may show a partial or total lack of structural organization and functional coordination with normal tissue. As used herein, a tumor is intended to encompass hematopoietic tumors as well as solid tumors.

A tumor may be benign (benign tumor) or malignant (malignant tumor or cancer). Malignant tumors can be broadly classified into three major types. Malignant tumors arising from epithelial structures are called carcinomas; malignant tumors that originate from connective tissues such as muscle, cartilage, fat, or bone are called sarcomas; and malignant tumors affecting hematopoietic structures (structures pertaining to the formation of blood cells) including components of the imnune system are called leukemias and lynphomas, Other tumors include, but are not limited to, neurofibromatoses.

Proliferative disorders include all types of cancerous growths or oncogenic processss, metastatic tissues or malignantly transformed cells, tissues, or organs, irrespective of histopathologic type or stage of invasiveness. Cancers include malignancies of various organ systems, such as the lung, breast, thyroid, lymphoid, gastrointestinal, and genito-urinary tract, as well as adenocarcinomas, which include malignancies such as most colon cancers, renal-cell carcinoma, prostate cancer and/or testicular tumors, non-small cell carcinoma of the lung, cancer of the small intestine and cancer of the esophagus. Carcinomas include malignancies of epithelial or endocrine tissues, such as respiratory system carcinomas, gastrointestinal system carcinomas, genitourinary system carcinomas, testicular carcinomas, breast carcinomas, prostatic carcinomas, endocrine system carcinomas, and melanomas. Other carcinomas include those forming from tissue of the cervix, lung, head and neck, colon and ovary. Cancers of the central nervous system include gliomas, (including astrocytomas, mixed oligoastrocytomas, glioblastoma multiform, ependymoma, and oligodendroglioma), meningiomas, pituitary tumors, hemangioblastomas, acoustic neuromas, pineal gland tumors, spinal cord tumors, hematopoietic tumors, and central nervous system lymphomas. Cancers effecting connective tissue, such as fat, muscle, blood vessels, deep skin tissues, nerves, bones, and cartilage are called sarcomas. Sarcoma include, for example, liposarcomas, leiomyosarcomas, rhabdomyosarcomas, synovial sarcomas, angiosarcomas, fibrosarcomas, neurofibrosarcomas, Gastrointestinal Stromal Tumors (GISTs), desmoid tumors, Ewing's sarcomas, osteosarcomas, and chondrosarcomas.

The methods described herein are particularly relevant for the treatment of humans having an epithelial malignancy, such as a lung cancer (*e.g.,* non~small~cell lung cancer (NSCLC)), breast cancer, colorectal cancer, head and neck cancer, or ovarian cancer. Epithelial malignancies arc cancers that effect epithelial tissues,

A "subject" as described herein can be any subject having a proliferative disorder, For example, the subject can be any mammal, such as a human, including a human cancer patient. Exemplary nonhuman mammals include a nonhuman primate (such as a monkey or ape), a mouse, rat, goat, cow, bull, pig, horse, sheep, wild boar, sea otter, cat, and dog. The methods described herein can be performed on any subject of any age, including a fetus (*e.g., in utero*)*,* infant, toddler, adolescent, adult, or elderly human. In some embodiments, the subject has an epithelial malignancy; a lung cancer, e.g., non-small cell lung cancer; breast cancer; colorectal cancer; head and neck cancer; or ovarian cancer.

An "antimetabolite" as used herein is a chemical with a similar structure to a substance (a metabolite) required for normal biochemical reactions, yet different enough to interfere with the normal functions of cells. Antimetabolites include purine and pyrimidine analogs that interfere with DNA synthesis. Exemplary antimetabolites include, *e*.*g*., aminopterin, 2-chlorodeoxyadenosine, cytosine arabinoside (ara C), cytaribine, fludarabine, fluorouracil (5-FU) (and its derivatives, which include capecitabine and tegafur), gemcitabine, methopterin, methotrexate, pemetrexed, raltitrexed, trimetrexate, 6-mercaptopurine, and 6-thioguanine.

An "antitubulin" as used herein refers to a chemotherapeutic agent that bocks cell division by inhibiting the mitotic spindle. Antibulin agents include, for example, the taxanes paclitaxel and docetaxel, and the vinca alkaloids vinorelbine, vincristine, vinblastine, vinflunine, and vindesine.

A "platinum-containing agent" as used herein includes chemotherapeutic agents that contain platinum. Platinum-containing agents cross-link with and alkylate DNA, which results in the inhibition of DNA synthesis and transcription. The platinum-containing agents can act in any cell cycle, and consequently kill neoplastic as well as healhy dividing cells. Platinum-containing agents include, for example, cisplatin carboplatin and oxaliplatin.

Unless otherwise defined, all technical and scientific terms used herein have the same meaning as commonly understood by one of ordinary skill in the art to which this invention belongs. Methods and material are described herein for use in its present invention; other, suitable methods and materials known in the art can also be used. The materials methods, and examples are illustrative only and not intended to be limiting. In case of confliet, the present specification, including defintions, will control.

Other features and advantages of the invention will be apparent from the following detailed description and figures, and from the claims.

### DESCRIPTION OF DRAWINGS

FIG. 1 is a Western blot of cell lines showing expression of TS. Cytosolic and nuclear extracts were prepared from cell lines H23-Ct and H23-R1. They were separated in polyacrylamide gels, transferred to membranes, and probed with antibodies directed to TS, Oct-1 (a nuclear protein), and GAPDH (a cytosolic protein). A band of 38 kD represeating TS was found in the cytoplasm.

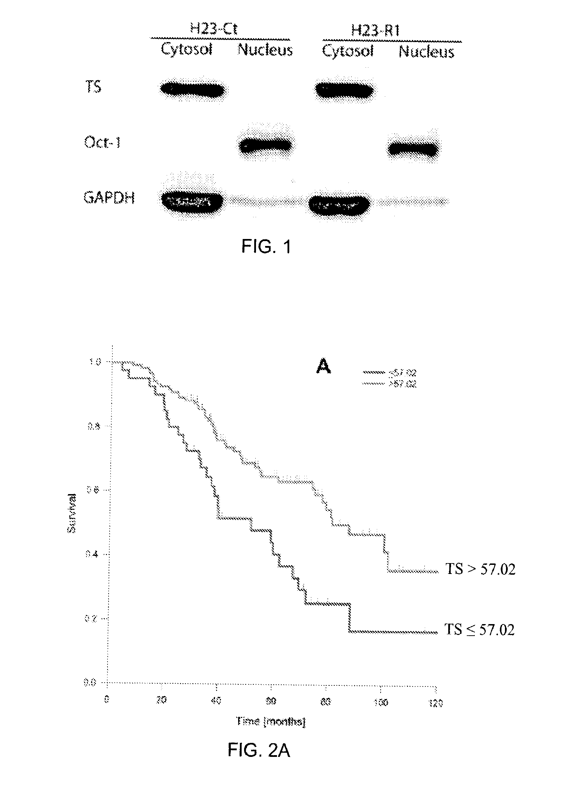

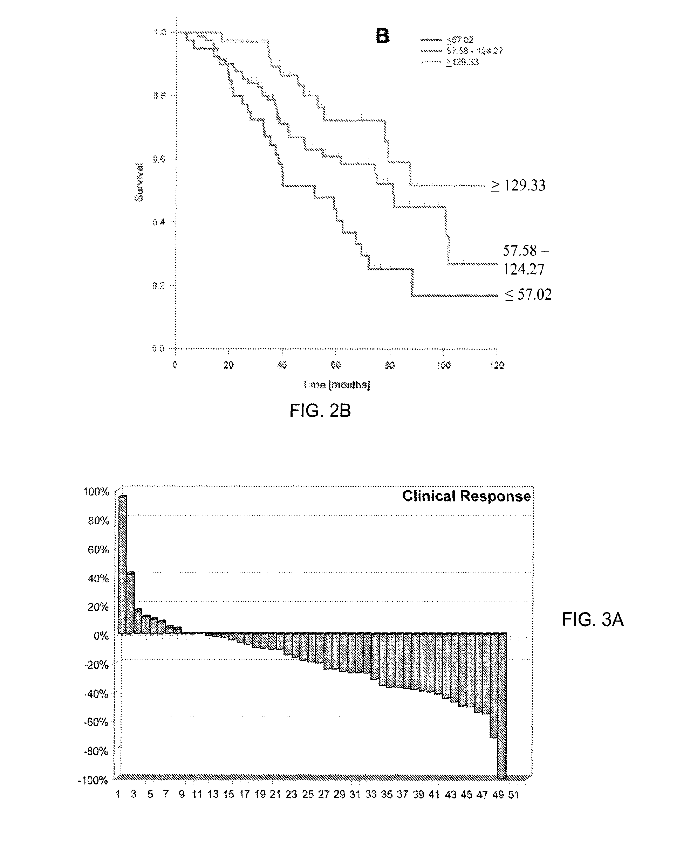

FIGs. 2A and 2B are Kaplan Meier overall survival curves, estimated by TS expression. 2A, Survival of 160 patients by *in situ* protein expression, Light grey lines depiet patients with marker expression> 57.02 (N=120; median=81.3 months, 95% CI 62.9-99.6), and dark grey lines depiet expression ≤ 57.02 (N=40; median=51.7 months, 95% CI21.5 -81.9). The unadjusted *p*-value was 0.0013. 2B, Survival of the same group of patients incorporating the second best cutpoint. Light grey lines depict patients with marker expression ≥ 129.33 (N=18; median not reached), medium grey depict patients with marker expression from 57.58 to 124.27 (N=82; median 80.8 months), and dark grey lines depiet expression ≤57.02 (N=40; median=51.7months). The unadjusted *p*-value was 0.002.
FIG. 3A is a bar graph showing the response to treatment as measured radiographically. The response ranged from a 95% increase to a 100% decrease in the size of measurable lesions
FIGs, 3B-E are bar graphs showing radiographic disease response and mRNA gene expression. 3B, RRM1; 3C, RRM2; 3D, TS; 3E, dihydrofolate reductase (DHFR).
FIG. 4 is a graph showing the results of Kaplan-Meier overall and disease-free survival estimates. The black curve (top line) denotes OS and the grey curve (bottom line) denotes DFS. Tick marks indicate censored cases.
FIG. 5 is a graph illustrating pre- and post-treatment mRNA levels of the genes RRMI and TS in 10 patients,

### DETAILED DESCRIPTION

A major goal of current research efforts in NSCLC is to increase the efficacy of perioperative systemic therapy in patients with a complete surgical resection through incorporation of molecular parameters into clinical therapeutic decisions. As described herein, intratumoral expression levels of the thymidylate synthase (TS) gene are correlated with clinical outcome, and levels of TS and Ribonucleotide Reductase M1 (RRMI) are correlated with tumor responsiveness to treatment with non-platinum antimetabolite drugs.

### Non Small Cell Lung Cancer (NSCLC)

Methods of diagnosing NSCLC are known in the art. Diagnostic procedures should be personalized for the individual patient and may include:
- Review of old chest X-rays to exclude long-standing benign lesions, or to determine rate of a malignant lesion.
- Sputum cytology
- Bronchoscopy, biopsy of endobronchial lesions, brushings and washings, post-bronchoscopy sputum cytology.
- Mediastinoscopy to exclude inoperable metastases in mediastinal lymph nodes
- Percutaneous fine needle biopsy
- Excisional or needle biopsy of readily accessible secondary deposits Histological or cytological verification of the diagnosis should be obtained.

Staging can also be performed using methods known in the art, e.g., the internationally-accepted UICC (International Union Against Cancer) TNM (Tumor/Node/Metastasis) classification system, as described in Mountain, Chest 111:1710-17 (1997); Pisters and Gail, J. Thoracic One 2(7):583-584 (2007); Rami-Porta et al., J.Thoracic One. 2(7):593-602 (2007); Rusch et al., J. Thoracic One. 2(7):603-612 (2007); Postumus et al., J. Thoracic One. 2(8):686-693 (2007); Groome et al., J. Thoracic One. 2(8):694-705 (2007); Goldstraw et al., J. Thoracic One. 2(8);706-714 (2007). Briefly, "T" describes the size of the tumor and whether it has invaded nearby tissue, "N" describes any lymph nodes that are involved, and "M" describes metastasis (spread of cancer from one body part to another). According to the TNM scale, stage 0 indicates carcinoma in situ, which is an cady cancer present only in the layer of cells in which it began. Stages I, II, III, and IV indicate progressively worsening disease states. Higher stages indicate more extensive disease as evidenced by greater tumor size and/or spread of the cancer to nearby lymph nodes and/or organs adjacent to the primary tumor. In stage IV, the tumor has spread to at least one other organ.

### Methods of Prognosis

As described herein, levels of TS protein, e.g., cytoplasmic TS protein, can be used to determine a prognosis, e,g., to determine likelihood of survival over an extended period, In these methods, a sample comprising tumor cells is taken from the subject, and levels of TS protein are determined using methods known in the art, e.g., as described herein. For example, its protein expression levels can be determined using a method of quantitative *in situ* analysis of protein expression, e.g., by automated quantitative analysis (AQUA), see, e.g., Camp et al., Nat. Med. 8:1323.-1327 (2002). Comparison of the levels of TS in the tumor cells in the sample to a reference level is indicative of the subject's likely outcome, In some embodiments, the comparison is made to a cutpoint percentile(s) determined by TS protein expression in a reference cohort as described herein. In some embodiments, the reference cutpoint is a level of expression that is about the 89th percentile; subjects above that cutpoint, e.g., in the highest 11% of expression levels, have an improved chance of survival, and subjects below that outpoint have a poorer prognosis. In some embodiments, two reference cutpoints are used, at a lower 25% group, middle 64% group, and upper 11% group; subjects falling into the lowest 25% have the highest risk of adverse outcome (death), subjects in the middle 64% have a medium risk level, and subjects in the highest 11% have a lower risk. See, e.g., Example and figures 2A-B. In some embodiments, a decision regarding how aggressively to treat a subject is made based upon the prognostic information provided by these methods, e.g., a subject with a higher risk of adverse outcome is treated more aggressively, or with the administration of additional treatment modalities, e.g., radiation or other chemotherapeutic agents as described herein.

### Methods of Determining Cancer Therapy

Methods of determining or selecting an appropriate cancer therapy include obtaining or providing a tumor sample from a patient, and determining the level of expression of RRM1 or TS in the patient. If intratumoral levels of RRM1 and TS are low, it can be determined that a chemotherapy containing a combination of gemcitabine and pemetrexed is appropriate. If intratumoral levels of RRM1 and/or are high, it can be determined that a chemotherapy lacking gemcitabine and pemetrexed is appropriate.

"Low" and "high" expression levels are relative values and are based on a comparison with those of a reference cohort. A "reference cohort," as used herein, is a sample cancer population from which RRM1 and/or TS expression data is collected. The expression level in a reference cohort is determined by measuring intratumoral gene expression levels in the sample population (see, e.g., Rosell et al., Clin Cancer Res 10:1318-25, 2004; Lord et al., Clin Cancer Res 8:2286-2291, 2002; Bepler et al., J Clin Oncol 22:1878-85, 2004; and Simon et al., Chest 127:978-83, 2005). Typically, a tumor exhibits "low" RRM1 levels if the expression level is equal to or less than the median RRM1 expression level in the reference cohort, and the tumor exhibits "high" RRM1 levels if the expression level is greater than the median RRM1 expression level in the reference cohort. Similarly, a tumor exhibits "low" TS levels if the expression level is equal to or less than the median TS expression level in the reference cohort. "Low" and "high" expression levels are relative and can be established with each new reference group. In one alternative, the expression level determined to be predictive of a subject's response to a chemotherapy can be equal to or less than the expression level of the lowest third, or lowest quartile of a reference cohort, or the predictive expression level can be determined to be a level equal to or greater than the expression level of the highest third, or highest quartile of a reference cohort.

### Tumor Samples

Any method can be used to obtain a tumor sample, such as a biopsy (*e.g*., core needle biopsy), and the tissue can be embedded in OCT^{®} (Optimal Tissue Cutting compound) for processing. For example, the tissue in OCT^{®} can be processed as frozen sections. Tumor cells can be collected, such as by laser capture microdissection (LCM), and gene expression can be assayed by, for example, reverse transcription coupled to polymerase chain reaction (RT-PCR) or Northern blot analysis to measure RNA levels, or by Western blot, to measure protein levels. In one exemplary approach, the level of RRM1 or TS expression is assayed by real-time quantitative RT-PCR. The level of expression of these genes can also be determined by immunohistochemistry, e.g., in fixed specimens, e.g., fixed with formalin.

### Reference Cohort

The samples from a reference cohort are taken from subjects of the same species (e.g., human subjects), and the tumors of a reference cohort are preferably of the same type (e.g., tumors of a NSCLC). For example, the tumors of a reference cohort can all be, for example, carcinomas, hematopoietic tumors, brain tumors, or sarcomas. In some embodiments, the tumors of a reference cohort can all be, for example, from a lung cancer, a breast cancer, a colorectal cancer, a head and neck cancer, or an ovarian cancer. The individual members of a reference cohort may also share other similarities, such as similarities in stage of disease, previous treatment regimens, lifestyle (*e,g*., smokers or nonsmokers, overweight or underweight), or other demographics (e.g., age, genetic disposition). For example, besides having the same type of tumor, patients in a reference cohort may not have received any previous systemic chemotherapy. A reference cohort should include gene expression analysis data from tumor samples from at least 10 subjects, e.g., from 15, 20, 25, 30, 40, 50, 60, 70, 80,90, 100, 120, 140, 160, 180, or 200 or more subjects.

### Determining Gene Expression and Protein Levels

Gene expression levels in a reference cohort can be determined by any method, such as by quantitative RT-PCR, Northern blot analysis, Western blot analysis, or immunohistochemistry. Expression levels in a tumor sample from a test subject are determined in the same manner has expression levels in the reference cohort,

Sequences useful in the present methods, when practiced in humans, include, but are not limited to the following:
- RRM1 sequences: GeneID: 6240 - human RRM1 - NM_001033.3 and NP_001024.1; gene = NC_000011.8 Reference assembly, Range 4072500-41 16682: NT_009237.17, Range 2903165-2947347
- TS sequences GenelD: 7298 - human TS NM_001071.2 and NP_001062.1; gene=NC_00018.8 Reference assembly - Range 647651-663492; NT_010859.14, Range 647651-663492.

Where it is desirable to determine protein levels, e.g., using Western Blotting, the following antibodies can be used; others may also be useful.
- anti∼RRM1 commercially available from Santa Cruz Biotechnology, Inc., Novas Biologicals, Proteintech Group, Inc., and Abnova Corporation, *inter alia.*
- anti TS commercially, available from Abeam, Serotec, Acris Antibodies GmbH, Invitrogen, Lab Vision, Millipore Corporation, Novus Biologicals, ProSci, Inc., Rockland Immunochemicals, Inc. and Santa Cruz Biotechnology, Inc., *inter alia.*

### Selection of a Therapeutic Regimen

The tumor can be sampled for expression levels of RRM1 or TS or both, and an appropriate chemotherapy can be determined based on the observed expression levels. The chemotherapy can include a single agent or multiple chemotherapeutic agents (*e.g.,* two, three, or more chemotherapeutic agents).

In one example, intratumoral expression levels of both RRM1 and TS are determined, and an appropriate chemotherapeutic agent is determined based on the expression level of both genes. For example, if RRM1 and TS expression levels are both determined to be low, an appropriate chemotherapy can be selected that includes a combination of gemcitabine and pemetrexed. If RRM1 levels are dermined to be low and TS levels are determined to be high, an appropriate chemotherapy can be determined to include gemcitabine and to exclude pemetrexed, and an optional second agent, such as an antitubulin or alkylating agent may be included. Such a chemotherapeutic composition could include a platinum-containing agent. If RRM1 levels are determined to be high and TS levels are determined to be low, an appropriate chemotherapy should not include gemcitabine but may include pemetrexed, and an optional second agent, such as an antitubulin or an alkylating agent. If RRM1 and TS levels are both determined to be high, an appropriate chemotherapy can be determined to include an antitubulin. The chemotherapy should not include an antimetabolite.

### Additional Treatments

In general, subjects diagnosed with NSCLC are treated with surgical resection; radiation; and adjuvant and/or neoadjuvant chemotherapies, see, e.g., Kris et al, Oncologist 10 (Suppl.2):23-29 (2005). The current standard of care for treating NSCLC is surgical resection, when feasible, followed by adjuvant chemotherapy in stages II and III; see Allen and Jahanzeb, J. Natl. Compr.Cane.Netw. 6(3):285-293 Also used are trimodal approaches involving the concurrent or sequential addition of radiotherapy. Thus,the methods described herein can include the use of any additional treatment modality, e.g., surgical resection and/or radiation.

Other chemotherapeutic agents can also be administered with the antimetabolite combination, e.g., antitubulin or platinum-containing agents. Other chemotherapeutic agents include, for example, L-asparaginase, bicalatamide, bleomycin, camptothecin (CPT-11), carminomyein, cyclophosphamide, cytosine arabinoside, dacarbazine, dactinomycin, doxorobicin, daunorubiein, ecteinascidin 743, estramustine,etoposide, etoposide phosphate, epothilone, flutamide,FK506,hexamethyl melamine, idatrexate, leflunimide, leuprolide, leurosidine, leurosine, melphalan, mitomycin C, mycophenolate mofetil, plicamycin, podophyllotoxin, porfiromycin, ranpimase, rapamyein, topotecan, teniposide, and thiotepa.

### Dosing and Administration

A chemotherapy, e.g., the pemetrexed/gemcitabine combination therapy selected for the subject based on RRM1 and/or TS expression levels as described herein, can be administered to a subject using conventional dosing regimens.

Chemotherapy can he administered by standard methods, including orally, such as in the form of at pill, intravenously, by injection into a body cavity (such as the bladder), intramuscularly, or intrathecally. A chemotherapy regimen can be delivered as a continuous regimen, *e.g*., intravenously, orally, or in a body cavity. A chemotherapy regimen can be delivered if a cycle including the day or days the drug is administered followed by a rest and recovery period. The recovery period can last for one, two, three, or four weeks or more, and then the cycle can be repeated. A course of chemotherapy can include at least two to 12 cycles (*e.g*.,three, four, five, six, seven, ten or twelve cycles).

Gene expression data obtained from the methods featured herein can be combined with information from a patient's medical records, including demographic data; vital status; education; history of alcohol, tobacco and drug abuse; medical history; and documented treatment to adjust conclusions relating to the prognosis of a proliferative disorder following administration of a chemotherapy designed as described above,

Upon administration of a chemotherapy according to the intratumoral RRM1 or TS expression levels, patient can be monitored for a response to the therapy. For example, tumor measurements can be taken before and after administration of the chemotherapy to monitor disease progression. If tumor size decreases, the disease can be determinedto be in remission, of regressing towards remission. A partial decrease in tumor size can indicate a disease in partial remission, and if the tumor completely disappears, the disease can be said to be in complete remission. If tumor size increases, the disease can be determined to be progressing. If tumor size does not change following administration of the chemotherapy, the disease can be categorized as stable.

A subject can also be assessed according to his physical condition, with attention to factors such as weight loss,pleural effusion, and other symptoms related to the cancer. For example, symptoms of lungs cancer, including small-cell and non-small cell lung carcinoma include persistent cough, sputum streaked with blood, chest pain_{,} and recurring pneumonia or bronchitis. The assessment can be used to tailor the dosage and administration regimen of the combination therapy according to methods known in the art.

### Methods of Predicting Efficacy and Screening Methods

Also described are methods of assessing or predicting the efficacy of a composition containing a chemotherapeutic agent. The methods employ at least two cell lines and a composition containing one or more chemotherapeutic agents. The cell lines differ in their level of expression of RRM1 and/or TS. For example, one cell line expresses a lower level of RRM1 than a standard control cell line, or one cell line expresses a higher level of RRM1 than a standard control cell line. The higher-expression cell line preferably expresses at least about 20%, 40%, 60%, 80%, 100%, 200% or 300% more RRM1 and/or TS than one lower-expression cell line.

Any manner of causing increased or decreased expression of RRM1 and/or TS can be utilized. For example, a cell line expressing a lower level of RRM1 and/or TS can be engineered to express an siRNA or antisense RNA that causes the lower level of expression. Alternatively, RRM1 and/or TS expression can be placed under control of a regulatable promoter, such as a tetracycline-, IPTG-, or ecdysone-responsive promoter. RRM1 and/or TS expression may be lower than expression in a parent strain, or expression may be completely absent prior to induction. Cells expressing high levels of RRM1 and/orTS can contain an RRM1 and/or TS gene under control of a constitutive promoter that expresses RRM1 and/or TS at a higher level than the endogenous RRM1 and/or TS promoter, or RRM1 and/or TS can be expressed from an inducible promoter, such as a tetracycline- or IPTG-responsive promoter, such that induction drives expression to a greater level than that in the parent strain. An exogenous sequence that drives a higher level of RRM1 and/or TS expression, or directs a lower level of expression can be stably integrated into the genome of the parent strain, or can be transiently transfected into the parent strain.

Cells that express high and low levels of RRM1 and/or TS are contacted with a candidate chemotherapeutic agent or a combination of chemotherapeutic agents *(e.g.,* 2, 3, or 4 chemotherapeutic agents), and the cells are monitored for an increased sensitivity or resistance to the chemotherapeutic agent or agents as compared to a control strain. An agent, or combination of agents, that causes an increased sensitivity to one cell line over a control cell line can be identified as a candidate therapeutic agent for a patient who has a tumor expressing the corresponding level of RRM1 and/or TS.

In another example, if cells expressing low levels of TS (*i.e.,* lower levels than a control parent strain) are more sensitive to a chemotherapeutic agent than the cells of the control strain, then the agent is a candidate therapeutic agent for the treatment of a patient with a tumor expressing low levels of TS. If cells expressing high levels of TS (*i.e.,* higher levels than a control parent strain) are more sensitive to a chemotherapeutic agent than the cells of the control strain, then the agent is a candidate therapeutic agent for the treatment of a patient with a tumor expressing high levels of TS.

The methods described herein can be used in screening assays to identify agents that are candidates for the treatment of tumors expressing high or low levels of RRM1 and/or TS. Cell lines such as those described above can be contacted with a panel of agents (*e.g.,* small molecule drugs, nucleic acids, or polypeptides) to identify agents that cause increased sensitivity of cells expressing low or high levels of RRM1 and/or TS

Agents identified in the above screening methods, or agents or combinations of agents identified by the above methods as candidate chemotherapies can be tested in animal models before being tested in humans. For example, the therapies can be tested for the ability to reduce tumor size in mice or primate models, before testing in humans.

### Kits

Reagents, tools, and/or instructions for performing the methods described herein can be provided in a kit. For example, the kit can contain reagents, tools, and instructions for determining an appropriate therapy for a cancer patient. Such a kit can include reagents for collecting a tissue sample from a patient, such as by biopsy, and reagents for processing the tissue. The kit can also include one or more reagents for performing a gene expression analysis, such as reagents for performing RT-PCR, Northern blot, Western blot analysis, or immunohistochemistry to determine RRM1 and/or TS expression levels in a tumor sample of a human. For example, primers for performing RT-PCR, probes for performing Northern blot analyses, and/or antibodies for performing Western blot and immunohistochemistry analyses can be included in such kits. Appropriate buffers for the assays can also be included. Detection reagents required for any of these assays can also be included.

The kits featured herein can also include an instruction sheet describing how to perform the assays for measuring gene expression. The instruction sheet can also include instructions for how to determine a reference cohort, including how to determine RRM1 and/or TS expression levels in the reference cohort and how to assemble the expression data to establish a reference for comparison to a test subject. The instruction sheet can also include instructions for assaying gene expression in a test subject and for comparing the expression level with the expression in the reference cohort to subsequently determine the appropriate chemotherapy for the test patient. Methods for determining the appropriate chemotherapy are described above and can be described in detail in the instruction sheet.

In another example, a kit featured in the invention can contain reagents, tools, and instructions for predicting the efficacy of a candidate chemotherapeutic agent based on RRM1 or TS expression levels. Such a kit can include vectors for modulating RRM1 or TS expression levels in a cell, and reagents for monitoring cell phenotype, such as reagents for detecting apoptosis. Reagents for determining the expression levels of RRM1 and TS in the tissue samples can also be included as described above.

Informational material included in the kits can be descriptive, instructional, marketing or other material that relates to the methods described herein and/or the use of the reagents for the methods described herein. For example, the informational material of the kit can contain contact information, *e.g.,* a physical address, email address, website, or telephone number, where a user of the kit can obtain substantive information about performing a gene expression analysis and interpreting the results, particularly as they apply to a human's likelihood of having a positive response to a specific chemotherapy,

A kit can contain separate containers, dividers or compartments for the reagents and informational material. A container can be labeled for use for the determination of RRM1 and TS gene expression levels and the subsequent determination of an appropriate chemotherapy for the human.

The informational material of the kits is not limited in its form. In many cases, the informational material, *e.g.,* instructions, is provided in printed matter, *e.g.,* a printed text, drawing, and/or photograph, *e.g.,* a label or printed sheet. However, the informational material can also be provided in other formats, such as Braille, computer readable material, video recording, or audio recording. Of course, the informational material can also be provided in any combination of formats.

### EXAMPLES

The invention is further described in the following examples, which do not limit the scope of the invention described in the claims.

### Example 1 - In situ Thymidylate Synthase (TS) Protein Expression

Thymidylate synthase (TS) catalyzes the conversion of deoxyuridine mono-phosphate (dUMP) to (deoxy)thymidine mono-phosphate (TMP), which requires oxidatation of tetrahydrofolate to dihydrofolate. TMP is subsequently phosphorylated to TTP, which is required for DNA synthesis and repair, 5-fluorouracil (5FU) inhibits TMP synthesis and is an effective chemotherapeutic agent (Washtien, Mol Pharmacol 1934;25:171-77; Moertel et al., N Engl J Med 1990;322:352-58; Heidelberger et al., Nature 1957;179(4561):663-6). High tumoral levels of TS have been associated with resistance to 5FU-based chemotherapy, particularly in patients with colorectal carcinoma (CRC) and gastric carcinoma (Johnston et al., Cancer Res 1995;55(7):1407-12; Leichman et al., J Clin Oncol 1997;15(10):3223-9: Shirota et al., J Clin Oncol 2001;19(23):4298-304).

Since TS is down-stream of ribonucleotide reductase and crucial for the formation of one of the deoxynucleotides required for DNA synthesis and repair, the present study was conducted to determine whether TS expression at the protein and mRNA levels is prognostic of outcome in patients with completely resected stage INSCLC who did not receive additional chemotherapy or radiation and if *in situ* TS expression is correlated with RRM1 and ERCC1 expression.

The two populations used in these studies consisted of patients that underwent a complete resection of NSCLC at the Moffitt Cancer Center from 1991 to 2001 and has been described elsewhere (Zheng et al., N Engl J Med 2007;356:800-08). In brief, patients had to have pathological stage IA or IB disease; adenocarcinoma, squamous cell carcinoma, or large cell carcinoma; no perioperative chemotherapy or radiation; no prior lung cancer, and no prior radiation to the chest. 187 were identified patients with sufficient tumor tissue for construction of tissue microarrays that comprised the population for assessment of gene expression at the protein level (Table 1), 92 patients with fresh frozen tumor tissue comprised the population for assessment of gene expression at the mRNA levels (Table 2). Thirty-two patients with gene expression values at the protein and mRNA levels overlapped between the two populations. Follow-up was recommended as three-monthly visits for two years, six-monthly visits for three years, and then annual visits. For overall survival, the time from diagnosis to death was recorded and verified using vital statistics records.

The monoclonal TS antibody used for the study was commercially available through Lab Vision Corporation (order #MS-471-P, lot #471P504B). It was generated in mice using recombinant human TS as the antigen. The antibody delected a dominant band of approximately 38 kD, the expected molecular mass of TS, with cytoplasmic localization on Western blots of lysates of lung cancer cell line H23 (Fig. 1). Western blotting was performed as follows. Cytoplasmic and nuclear extracts from permanent genetically modified cultures of cell line NCI-H23 were prepared using a nuclear/cytosol fractionation kit (BioVision, Mountain View, CA). Protein extracts (50 µg) were separated through 10% Novex tris-glycine gels (Invitrogen, Carlsbad, CA) and blotted onto pure nitrocellulose membranes (Bio-Rad Laboratories, Hercules, CA). The blots were incubated with TS antibody (mouse clone TS-106, 1:200, Lab Vision Corp.), Oct-I antiserum (#3342-100, 1:1000, BioVision, Mountain View, CA), and CAPDH antiserum (#sc-20357, 1:1000, Santa Cruz Biotechnology, Santa Cruz, CA) at 4°C overnight. Protein bands were visualized with anti-rabbit, anti-mouse, or anti-goat IgG horseradish peroxidase secondary antibody (1: 1000; Santa Cruz) and SuperSignal West Pico chemiluminescence substrate (Pierce, Rockford, IL). The house-keeping gene GAPDH was used as equal loading control.

Confocal microscopy was used to determine subcellular localization of TS protein, as follows. Lung adenocarcinoma cell lines (H23, H125, H292, H322, A549) and colon carcinoma cell lines (H498, H508, H747, SNU-C2A, SNU-C4) were grown directly on Lab-Tek chamber slides. Adherent cells were washed is phosphate buffered saline (PBS), fixed by incubation for 20 min, in 4% paraformaldehyde in PBS, and washed in PBS. They were permeabilized for 1 hr. in 0.25% Triton-X100/PBS and washed in PBS. TS (1:100) antibodies were diluted in binding buffer (1 %BSA/0.1 %NP40/PBS), added to the chambers, and incubated for 1 hr. After washing in PBS, the slides were incubated for 45 min. with 1:500 dilutions of Alexa Fluor 555 anti-mouse IgG (Molecular Probes, InVitrogen, Eugene, OR). The slides were washed with PBS and covered using ProLong Gold antifade reagent with DAPI (Molecular Probes, InVitrogen). As negative controls, the same procedure was performed without primary antibody. Samples were viewed with an inverted Zeiss LSM 510 confocal microscope with a 63x /L20NA water immersion objective. Nuclei were visualized with DAPL Images were produced with dual photomultiplier detector and the LSM 5 version 3.2.0.115 software suite.

The results indicated that the predominant cytoplasmic localization of TS in 5 lung cancer cell lines was confirmed (Fig. 2A); however, nuclear TS expression was observed in all, and it was lowest in H23 and highest in A549. In contrast, in 5 cell lines derived from colon carcinoma, TS expression was predominantly nuclear.

To create tissue microarrays, tumor specimens were collected prospectively, fixed in neutral-buffered formalin (10% v/v), and completely embedded in paraffin wax. Whole tissue sections were H & E strained, and representative tumor areas were marked. Tissue cores with a diameter of 0.6 mm were punched and arrayed into a recipient block using a tissue arrayer (Beecher Instrument, Silver Spring, MD). Sections of 5 µm thickness were cut, transferred to 4x adhesive coated slides (Instrumedies, NJ), and exposed to UV light for 30 seconds to enhance adherence.

*In situ* TS protein expression was determined by AQUA in the tumor cytoplasm in two replicate tissue microarrays that encompassed a total of 187 patients with completely resected NSCLC that had not received perioperative chemotherapy or radiation.

Immunohistochemistry (IHC) based on immunofluorescence combined with automated quantitative analysis (AQUA) was used to assess in situ expression of the target molecules 13 Antigens were retrieved by microwave oven treatment for 15 minutes in 0.01 mol/L of Na-citrate at pH 6.0. The slides were blocked tor 30 minutes with 0.3% BSA and then incubated overnight at room temperature with the primary antibody (mouse clone TS-106, 1:30, #MS-471-P, Lab Vision Corp., Fremont, CA). For identification of carcinomatous cells, an antiserum to cytokeratin was used (rabbit antihuman pancytokeratin AE1/AE3, 1:200, #Z0622, Dako Cytomation). Slides were washed and incubated with two different secondary antibodies for 1 hr. (Envision® labeled polymer-HRP anti-mouse, # K4007, and Alexa 555 goat anti-rabbit, #A21429, 1:200, Dako Cytomation). For fluorescence amplification, slides were exposed to Cy5-Tyramide (1:50) for 10 minutes at room temperature. They were mounted with Prolong Gold antifade reagent with DAPI (4'-6'diamidino-2-phenylindole) mound solution. The final tissue microarray slides were scanned with SpotGrabber, and image data were analyzed with AQUA (PM-2000, HistoRx, New Haven, Connecticut). The lowest possible AQUA score is 0 and the highest is 255.

The results are shown in Fig. 2B. TS expression data were obtained on 160/187 (86%) patients. Duplicate expression data were not available for 27 specimens because the spots were absent on the section or had washed off during the processing. Average values ranged from 5.8 to 238.6 (on a scale from 0 - 255) with a median of 98.4 and a mean of 98.3 (standard deviation of 53.0). The data were near normally distributed, and a data transformation for normalization was not performed. There was a significant correlation in TS expression between replicate arrays (*r* = 0.599, *p* ≤ 0.001).

There was no statistically significant association between TS protein expression and tumor stage, histology, performance status, absence or presence of weight loss, smoking status, or gender (Table 1). The unidimensional tumor diameters in this patient cohort ranged from 0.5 to 10.8 cm and did not correlate significantly with TS expression (*r* = 0.051, *p* = 0.52). There was no significant correlation with the previously reported RRM1 and ERCC1 protein levels (*r* = 0.13 and ∼0.07 respectively, *p* > 0.1) (Zheng et al., N Engl J Med 2007;356:800-08).

**Table 1: Patient Characteristics, TS Protein Expression, and Survival**

| | **Patient Characteristics** | | | **TS by AQUA** | | |
|---|---|---|---|---|---|---|
| | N (187) | Median Overall Survival [months] | log rank *p*-value | Median | N (160) | Kruskal Wallis *p*-value |
| **Stage** | | | | | | |
| IA | 85 | 100.5 | .019 | 99.6 | 71 | .818 |
| IB | 102 | 62.2 | | 97.3 | 89 | |

| **Histology** | | | | | | |
|---|---|---|---|---|---|---|
| Adeno | 78 | 72.0 | .061 | 98.4 | 67 | .427 |
| BAC | 18 | 87.4 | | 84.6 | 17 | |
| Squamous | 68 | 101.8 | | 101.5 | 55 | |
| Large Cell | 23 | 41.8 | | 93.3 | 21 | |

| **Performance Status** | | | | | | |
|---|---|---|---|---|---|---|
| 0 | 128 | 79.0 | .034 | 99.8 | 109 | .191 |
| 1 | 48 | 52.7 | | 90.4 | 41 | |

| **Weight Loss**. >5% in 3 months | | | | | | |
|---|---|---|---|---|---|---|
| absent | 159 | 74.9 | .823 | 94.3 | 133 | .278 |
| present | 14 | 81.3 | | 102.9 | 14 | |

| **Smoking Status** | | | | | | |
|---|---|---|---|---|---|---|
| Never | 11 | NR* | .077 | 101.2 | 8 | .326 |
| quit | 113 | 77.9 | | 94.4 | 98 | |
| Active | 49 | 61.4 | | 105.0 | 41 | |

| **Gender** | | | | | | |
|---|---|---|---|---|---|---|
| Women | 86 | 81.3 | .143 | 103.6 | 73 | .284 |
| Men | 101 | 69.4 | | 91.6 | 87 | |

| | | | | | | |
|---|---|---|---|---|---|---|
| *NR, not reached | | | | | | |

Using the maximal log-rank method for optimal cutpoint determination, the most significant overall survival difference was seen if patients with levels of ≤57.02 were separated from those with levels >57.02, which was the 25^{th} percentile (40/160 patients) of TS expression (Fig. 3A). The median survival in the low TS group was 51.7 months. and it was 81.3 months for those in the high TS group (*p* = 0.0013). This survival difference remained significant after adjusting the *p*-value for multiple looks (*p* = 0.034).

In the final multivariate model, which included TS protein expression and tumor stage (Table 2), TS remained significantly associated with overall survival (*p* 0.0013, adjusted *p* = 0.032). The hazard ratio for death was 0.45 for high versus low TS protein expression.

**Table 2: Cox Regression Model**

| **Variable** | N | Univariate Hazard Ratio (95% CI*) | Univariate *p*-value | **Multivariate Hazard Ratio**** **(95% CI)** | **Multivariate** ***p*-value** |
|---|---|---|---|---|---|
| **TS group** **(low = reference)** | 160 | 0.46 (0.28 - 0.75) | 0.040** | **0.45** **(0.28 - 0.73)** | **0.032***** |
| **Stage** **(IA = reference)** | 160 | 1.72 (1.06 - 2.76) | 0.029 | **1.76** **(1.08 - 2.87)** | **0.022** |
| **Gender** **(female = reference)** | 160 | 1.23 (0.77 - 1.96) | 0.393 | | |
| **ERCC1 expression** | 160 | 0.994 (0.986 - 1.002) | 0.130 | | |
| **RRM1 expression** | 160 | 0.988 (0.975 - 1.002) | 0.095 | | |
| **Performance Status** **(0 = reference)** | 150 | 1.46 (0.87 - 2.44) | 0.156 | | |

| | | | | | |
|---|---|---|---|---|---|
| *CI. confidence interval **, based on 160 patients, since performance states was eliminated from the final multivariate analysis ***, adjusted p-value to account for optimal cutpoint selection | | | | | |

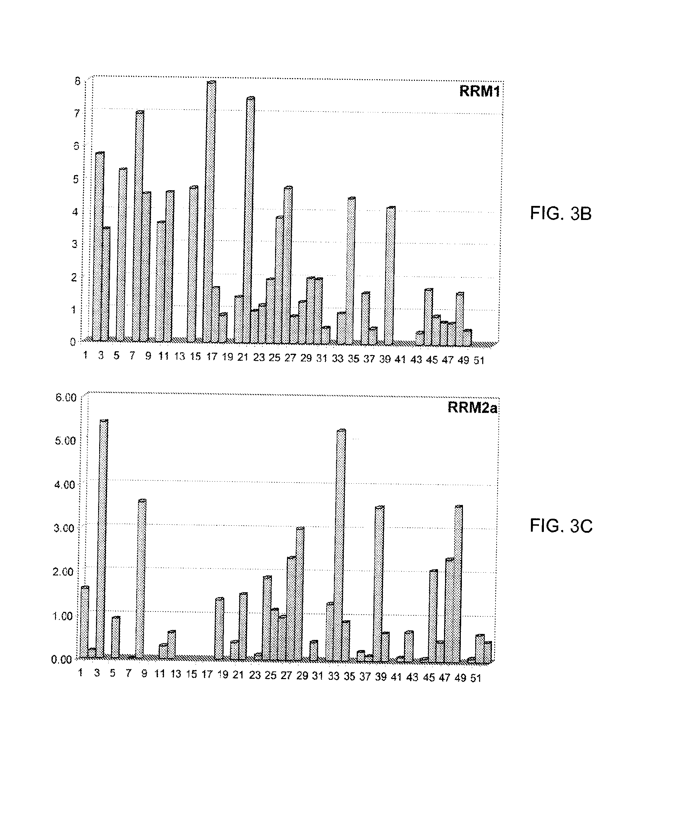

In addition to the planned survival analysis, exploratory examinations of the association of TS expression and survival were conducted. TS expression was included in a Cox regression analysis as a continuous variable and yielded low *p*-value both unadjusted (*p* = 0.0061 and adjusted for tumor stage (*p* = 0.002). A second cutpoint for TS protein expression was also found, with an AQUA score of 129.33 (89th percentile). Inclusion of this outpoint in the survival analysis yielded a classification of patients into three survival groups (Fig. 3B). The median survival in the highest TS group was >120 months, it was 80.9 months in the medium TS group and 51.7 months in the lowest TS group (*p* = 0.002).

A dichotomization of the TS expression data using the sample median of 98.4 as a cutpoint, revealed a median OS of 81.3 months for patients with high TS expression and 62.2 months for those with low TS expression (*p* = 0.071).

Statistical analysis in this example was performed as follows. The average values for AQUA scores from two replicate readings were calculated and treated as independent continuous variables. The average of TS mRNA expression was calculated from triplicate readings and was treated as an independent continuous variable. The primary objective was to assess the association between in situ TS protein expression and overall survival. For this TS expression was not classified into high and low categories a *priori.* Instead, the maximal log-rank method was used for optimal cutpoint determination and adjusted the p-values for multiple looks (Miller and Siegmund, Biometries 1982;38;1011-16; Hilsenbeck and Clark, Stat Med 1996;15(1):103-12). Outpoints within the central 80% of ordered TS protein expression were considered. The association between survival and low and high TS expression was then summarized in a Kaplan-Meier survival curve using the cutpoint percentile(s) determined by TS protein expression. A multivariate Cox regression analysis using backward elimination with a significance level-to-stay of 0.15 was performed to assess the impact of TS protein expression on survival while adjusting for the potential covariates tumor stage, performance status, gender, RRM1 protein expression, and ERCCI protein expression. RRM1 and ERCC1 protein expression were included as continuous variables. Since the dichotomised split for TS protein expression was obtained by optimal selection, its Cox regression p-value was adjusted. The maximal log-rank method for optimal cutpoint determination was also used to examine the association of TS mRNA expression and overall survival

Associations between continuous variables were analyzed using Spearman's correlation coefficient. Associations between continuous and discrete variables were analyzed using the Wilcoxon rank sum test or the Kruskal-Wallis test depending on the number of discrete groups. The log-rank test was used to assess survival differences between demographic and clinical groups,

Using a rigorous statistical approach to account for multiple testing, the results demonstrate that increased cytoplasmic TS expression was associated with prolonged survival. Two exploratory analyses supported this finding. Firstly, had we chosen *a priori* to look for a decrease in the risk of death with increasing TS levels by Cox regression analysis, a statistically significant result (*p* = 0.006) would have been obtained. Secondly, the risk of death shifts dramatically at two distinct levels of TS expression, 57.02 and 129.33. This survival model divided the patients into a lower 25% group, middle 64% group, and upper 11% group,

### Example 2 - TS mRNA expression

TS mRNA expression was determined in fresh-frozen tumor specimens from 92 patients with completely resected stage 1 NSCLC, as follows. Fresh-frozen tumor specimens had been prospectively collected on 92 patients that fulfilled the same selection criteria as described in Example 1 (Table 3).

**Table 3: Patient Characteristics. TS mRNA Expression, and Survival**

| | **Patient Characteristics** | | | **TS by RTPCR** | | |
|---|---|---|---|---|---|---|
| | N (92) | Median Overall Survival [months] | log rank *ρ*-value | Median | N (85) | Kuskal Wallis *ρ*-value |
| **Stage** | | | | | | |
| IA | 39 | NR* | .671 | 3.42 | 37 | .642 |
| IB | 53 | 74.9 | | 3.65 | 48 | |

| **Histology** | | | | | | |
|---|---|---|---|---|---|---|
| Adeno | 41 | 62.2 | .642 | 2.25 | 39 | .002 |
| BAC | 7 | 87.4 | | 0.50 | 6 | |
| Squamous | 33 | NR | | 4.73 | 30 | |
| Large Cell | 11 | 69.4 | | 5.08 | 10 | |

| **Performance Status** | | | | | | |
|---|---|---|---|---|---|---|
| 0 | 52 | 80.4 | .771 | 3.47 | 52 | .853 |
| 1-2 | 33 | 62 1 | | 3.87 | 26 | |

| **Weight Loss,** >5% in 3 months | | | | | | |
|---|---|---|---|---|---|---|
| absent | 75 | 80.4 | .020 | 3.52 | 69 | .703 |
| present | 6 | 34.1 | | 5.60 | 6 | |

| **Smoking Status** | | | | | | |
|---|---|---|---|---|---|---|
| Never | 6 | 31.2 | .898 | 4.53 | 4 | .057 |
| quit | 55 | 74.9 | | 2.26 | 51 | |
| Active | 27 | 88.2 | | 5.16 | 26 | |

| **Gender** | | | | | | |
|---|---|---|---|---|---|---|
| Women | 39 | 88.2 | .605 | 4.04 | 35 | .652 |
| Men | 53 | 74.9 | | 2.33 | 50 | |

| | | | | | | |
|---|---|---|---|---|---|---|
| * Nr, not reached | | | | | | |

RNA was isolated from tumor specimens with >60% of cells consisting of tumor and cDNA was generated using oligo-DT and random primers with reverse transcriptase (QuantiTect Reverse Transcription Kit, Qiagen, Valencia. CA). Approximately 5 ng of sample cDNA was used in triplicate for TS expression analysis by real-time technology (ABI 7900HT. Foster City, CA). The relative amount of TS mRNA in a sample was determined by comparing the threshold cycle with the standard curve, and the standardised amount was then determined by dividing the TS amount (ABI, HS00426591-ml, amplicon size 87 bp) by the 18SrRNA amount (ABI, #4319413E).

Data were obtained from 85 patients (92%), and expression ranged from 0.2 to 104.3 with a median of 3.5 and a mem of 6.7 (standard deviation of 12.9). mRNA levels were not significantly associated with tumor stage, performance status, absence or presence of weight loss, smoking status, or gender (Table 3). They were, however, significantly different among the histological subtypes; the levels were lower in adenocarcinomas compared to squamous and large cell carcinomas (Table 3). The unidimensional tumor diameters in this patient cohort ranged from 1.4 to 10.8 cm, and they did not correlate with *TS* expression (r = 0.165,*p* ::::: 0.218).

The maximal log-rank method was used for optimal cutpoint determination, and no statistically significant cutpoint for *TS* mRNA expression was found after adjusting for multiple looks (adjusted *p*::::1.0).

Thirty-two patients were common to both datasets and thus had TS expression values at both the protein and mRNA levels, There was a near zero correlation between the levels (*r*=0.028. *p*=0.877).

Statistical analysis was performed as described in Example 1.

These results, which failed to detect an association between TS protein and mRNA expression using methods specifically developed for quantitative analysis, are consistent with a more recent report demonstrating a lack of correlation between protein and mRNA levels in lung cancers for the majority of genes (Chen et al., Mol Cell Proteomics 2002; 1 (4):304-13) .

### Example 3 - Clinical Efficacy and Predictive Molecular Markers of Neoadjuvant Gerncitabine and Pemetrexed in Resectable Non-Small-Cell Lung Cancer

Incorporation of platinum-containing chemotherapy into the management of resectable non-small-cell lung cancer (NSCLC) has become the standard of care for patients with metastatic disease in Ni or N2 lymph nodes (The International Adjuvant Lung Cancer Trial Collaborative Group. N Engl J Med 2004; 350:351-360; Winton et al.,N Engl J Med 2005; 352:2589-2597: Douillard et al., Lancet Oncol 2006; 7:719-727). Neoadjuvant treatment results in response rates of approximately 33-64% (Depierre et al., J Clin Oncol 2002; 20:247-253; Scagliotti, Proc Am Soc Clin Oncol 2005; 23:626s; Gilligan et al., Lancet 2007; 369:1929-1937; Pisters et al, Proc Am Soc Clin Oncol 2007; 25:389s), and adjuvant therapy increases absolute overall survival by approximately 5-15% (The International Adjuvant Lung Cancer Trial Collaborative Group. N Engl J Med 2004; ,350:351-360: Winton et al., N Engl J Med 2005; 352:2589-2597; Douillard et al., Lancet Oncol 2006; 7:719-727). However, the approach of treating all patients with a platinum-containing regimen may have reached a plateau in terms of efficacy. In addition, there is signifîcant toxicity associated with this approach including a treatment related mortality of approximately 1-2% (The International Adjuvant Lung Cancer Trial Collaborative Group. N Engl J Med 2004; 350:351-360; Pisters et al., Proc Am Soc Clin Oncol 2007; 25:389s). The incorporation of molecular tumor characteristics into therapeutic decisions may improve efficacy and reduce toxicity.

This example describes a single-institution trial of neoadjuvant gemcitabine and pemeirexed in patients with resectable NSCLC with the goal to describe the clinical efficacy and tolerability of the chosen regimen and to investigate the predictive utility of mRNA expression of genes involved in the metabolism of these drugs on therapeutic efficacy

Induction chemotherapy and toxicity: A total of 52 eligible patients, 26 men and 26 women, between the ages 41 and 83 years (median 67 years), received at least one dose of chemotherapy. They were enrolled between April 2004 and April 2006, and their characteristics are described in Table 4.

Clinical staging was determined by physical examination, computed tomography of the chest and upper abdomen (CT), whole body FDG positron emission tomography (PET), magnetic resonance imaging of the brain (MRI), bronchoscopy, and mediastinoscopy. Histological confirmation of NSCLC; stage 1B-IIIA and selected IIIB (2 lesions in one lobe, T4); age>18 years; a performance status (PS) of 0-1; measurable disease by RECIST; and no prior therapy for lung cancer were required for eligibility. These criteria were met by 52 patients.

Preoperative chemotherapy with gemcitabine 1,500 mg/m2 followed by pemetrexed 500 mg/m2 was administered on days 1, 15, 29, and 43. Subsequent doses of chemotherapy were delayed or reduced for toxicity if appropriate. Patients received oral folate at a dose of 350-1,000 ug daily and subcutaneous vitamin B12 at a dose of 1,000 ug every 9 weeks starting one week before chemotherapy. All toxicities were graded according to the common toxicity criteria (CTC, version 3.0).

Following chemotherapy, CT and PET scans were repeated between days 50 and 63. Radiographic response was expressed as a continuous variable by calculating the percentage of change in the sum of all greatest tumor diameters comparing the post-treatment and pre-treatment CT scans (1-[sum post lesions/sum pre lesions] x 100) and also by RECIST as best overall response.

Patients with resectable disease had thoracotomy between days 64 and 77. The recommended surgery was lobectomy or pneumonectomy with mediastinal lymph node dissection. Segmentectomy or wedge resection was discouraged. Patients with unresectable disease and those with incomplete resections were treated at the discretion of their physician. All patients were followed at 3-monthly intervals +for 2 years with a CT.

Forty-two patients received all planned chemotherapy on time and without dose reduction. Three patients had dose delays without dose reductions. The reasons were a grade 3 lower extremity cellulitis, a grade 2 thrombocytopenia, and a grade 3 liver enzyme elevation. Seven patients had less than the intended 4 bi-weekly therapies. Three patients had only the first cycle because of grade 3 neutropenic fever with renal failure, a grade 3 febrile drug reaction, and one patient requested immediate surgery without having experienced treatment-related side effects. Three patients only had cycles I and 2. The reasons were a grade 3 lower extremity phlebitis in one patient and grade 3 fatigue in two. One patient died after cycle 3 from pneumonitis of a likely viral etiology; however, a possibly treatment-related etiology could not be excluded.

**Table 4: Patient Characteristics, Disease Response and Survival**

| | | | **Radiographie Response** **Rate¹** | | **Pathologie Response** **Rate²** | | **Median Overall Survival** | | **Median Disease-Free Survival³** | |
|---|---|---|---|---|---|---|---|---|---|---|
| **All Patients** | | N=52 | 35% | (17849) | 30% | (13/43) | >28.0 m | | >21.1 m | |
| **Age** median (range) 67 y (41-83) | | | | | | | | | | |
| | < 67 y | N-26 | 38% | (9/24) | 25% | (6/24) | >28.0 m | | >21.1 m | |
| | ≥ 67 y | N=26 | 32% | (8/25) | 33% | (7/21) | >28.0 m | *p*=0 86 | 21.0 m | *p*=0 40 |

| **Gender** | | | | | | | | | | |
|---|---|---|---|---|---|---|---|---|---|---|
| | women | N=26 | 32% | (8/25) | 27% | (6/22) | >28.0 m | | 21.1 m | |
| | men | N=26 | 38% | (9/25) | 33% | (7/21) | >28.0 m | *p=*0.78 | >21.1 m | *p*=0.90 |

| **Performance Status** | | | | | | | | | | |
|---|---|---|---|---|---|---|---|---|---|---|
| | zero | N=31 | 36% | (10/28) | 36% | (10/28) | >28.0 m | | >21.1 m | |
| | one | N=21 | 33% | (7/21) | 20% | (3/15) | 16.8 m | *p*=0.005 | >21.1 m | *p*=0.70 |
| **Weight Loss⁴** | | | | | | | | | | |
| | absent | N=47 | 34% | (15/44) | 31% | (12/39) | >28.0 m | | >21.1 m | |
| | present | N=5 | 40% | (2/5) | 25% | (1/4) | 16.1m | *p=*0.04 | 9.1 m | *p=*0.30 |

| **Smoking Status⁵** | | | | | | | | | | |
|---|---|---|---|---|---|---|---|---|---|---|
| | active | N=23 | 30% | (6/20) | 21%, | (4/19) | >28.0 | | 21.1 m | |
| | quit (>1y) | N=27 | 41% | (11/27) | 36% | (8/22) | >28.0 m | *p*=0.35 | >21.1 m | *p.*0 70 |
| | never smoker N=2 | | NA⁶ | (0/2) | NA | (1/2) | | | | |

| **Histopathology**⁷ | | | | | | | | | | |
|---|---|---|---|---|---|---|---|---|---|---|
| | squamous | N=19 | 39% | (7/18) | 22% | (4/18) | >28.0 m | | >21.1 m | |
| | non squamous | N=33 | 32% | (10/31) | 36% | (9/25) | >28.0 m | *p*=0.99 | >21.1 m | *p*=0.91 |

| **Stage** | | | | | | | | | | |
|---|---|---|---|---|---|---|---|---|---|---|
| | I | N=16 | 31% | (5/16) | 27% | (4/15) | >28.0 m | | 20.7 m | |
| | II | N=18 | 38% | (6/16) | 29% | (4/14) | >28.0 m | *p* 0.77 | >21.1 m | *p*=0.58 |
| | III⁸ | N=18 | 35% | (6/17) | 36% | (5/14) | >28.0 m | | >21.1 m | |

| **Tumor Resection** | | | | | | | | | | |
|---|---|---|---|---|---|---|---|---|---|---|
| | complete | N=40 | 37% | (14/38) | 33% | (13/39) | >28.0 m | | >21.1 m | |
| | incop./not done | N=12 | 27% | (3/11) | 0% | (0/4) | 12.9 m | *p*=0.22 | NA | *p.* =NA |

| **Response to Chemotherapy⁹** | | | | | | | | | | |
|---|---|---|---|---|---|---|---|---|---|---|
| | CR/PR | N=17 | NA | (17/17) | 38% | (6/16) | 27.4 m | | 18.2 m | |
| | SD | N=29 | NA | (0/29) | 24% | (6/25), | >28 0 m | *p*=0.67 | >21.1 m | *p*=0.24 |
| | PD | N=3 | NA | (0/3) | 0% | (0/1) | >28.0 m | | | |

| | | | | | | | | | | |
|---|---|---|---|---|---|---|---|---|---|---|
| Legend to Table 4: ¹not assessed in three patient; ²not assessed in nine patients; ³in the 40 patients with complete tumor resection; ⁴equal to or greater than 5% during the three months prior to diagnosis; ⁵a life-time never smoker was defined as a person that had smoked less than a total of 100 cigarettes; ⁶NA, not applicable; ⁷21 patients had adenocarcinoma including 5 with bronchioloalveolar features, 12 had other NSCLC subtypes including 2 adenosquamous carcinomas, 2 mixed adeno∼ and squamous carcinomas, and 1 large cell carcinoma with suggestive neuroendocrine features; ⁸two T4N0, T4 because of two separate tumor nodules in a single lobe of the lung; ⁹the pathologie disease response categories pPR and pNR were not significantly associated with OS (hazard ratio for pPR vs. pNR = 1.1,*p* = 0.90) or DFS (hazard ratio for pPR vs. pNR = 0.87, *p* = 0.78) | | | | | | | | | | |

Radiographic response to chemotherapy: A radiographic response evaluation was possible in 49 patients. The best overall response was a compete remission (CR) in 1 (2% 95% CI: 01-10.9%patient; it was a partial remission (PR) in 16 (33%; 95% CI: 20.0-47.5%), stable disease (SD) in 29 (59%; 95% CI: 44.2-73.0%), and progressive disease (PD) in 3 (6%; 95% CI: 1.3-16.9%) patients. The response ranged from a 95% increase to a 100% decrease in the size of measurable lesions (Figure 3A). There were no statistically significant associations between radiographic disease response and the clinical parameters age *(p* = 0.62), gender (*p* = 0.96), performance status (*p* = 0.94), weight loss (*p* = 0.88), smoking status (*p* = 0.85), tumor histology (*p*= 0.95), and stage (*p* 0.31) (Table 4).

Pathologic response to chemotherapy: A pathologic response evaluation was performed by determination of the proportion of necrotic and/or fibrotic material on light microscopical evaluation of surgical resection specimens stained with H & E, This was possible in 43 patients, and it ranged from 0 -90%. None of the patients had a pathological CR (≥95% necrosis/fibrosis), 13 (30%) had a pathological PR (50-94% necrosis/fibrosis and 30 (70%) had no pathological response (pNR). There were no statistically significant associations between pathologic disease response and the clinical parameters age (*p* = 0.39), gender (*p* = 0,65 ), performance status (*p* =0.18), weight loss (*p* = 0.69), smoking status (*p* =0.96), tumor histology (*p* = 0.43), and stage (*p =* 0.66). Although there was it correlation between radiographic and pathologic response (Spearman's rho = 0.23i.e., better clinical response was associated with a higher proportion of necrosis and fibrosis_{,} it was not statistically significant (*p* = 0.14).

Surgical treatment: A thoracotomy was performed in 46 patients, and it resulted in a complete resection if 40 patients. Thirty-two patients had a lobectomy, 2 had a bilobectomy, 8 had a pneumonectomy, 2 had a wedge resection, and 2 had no tumor resection. At the surgical resection, 17 patients were down-staged, 17 had no change, and 12 were up-staged compared to the initial staging. One patient died 2.7 months after a complete right upper and middle lobectomy for a T2N1 squamous cell carcinoma of a bronchopleural fistula.

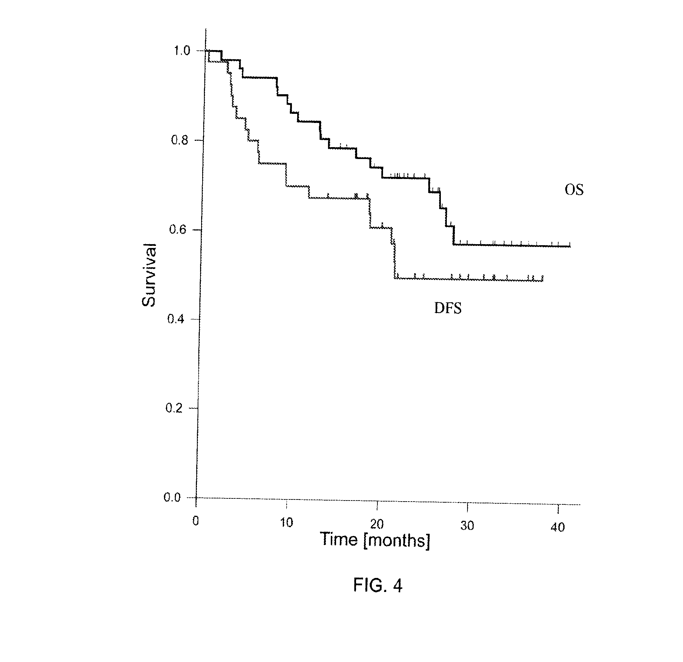

Survival: As of February 8 , 2008, 20 events had occurred and 32 patients were alive (5 with a recurrence, and 5 with an incomplete resection), The median OS was >28.0 months, and the median DFS for patients with a complete surgical resection was >21.1 months (Figure 4). The 12-month and 24-month OS rates were 84.6% (95% CI: 71.6- 92,0%) and 71.0% (95% CI: 56.5 - 81.4%), and the corresponding DFS rates were 67.5% (95% CI: 50.7- 79.7%) and 53.0% (95% CI: 36.0 - 67.4%) respectively. The radiographic or pathologic response to chemotherapy was not significantly associated with OS or DFS (Table 4).

Pharmacogenomic variables predictive of disease response: Pretreatment tumor specimens of sufficient quantity and quality for gene expression analysis by real-time RTPCR were available on 10 and post-treatment specimens on 35 patients. It was evaluated if gemcitabine and pemetrexed therapy would alter the mRNA levels of *RRMI* and *TS*.

Tumor samples were collected prior to and after therapy as frozen specimens. The standard operating procedure for collection included a recording of the time from biopsy or resection to freezing, and the time elapsed was 30 min or less in all cases. Frozen specimens were embedded in OCT and cut in 5-7 µm sections.

Tumor cells were collected by laser capture microdissection (LCM) using the Arcturus system. Total RNA was extracted using a commercial method (Arcturus, Mountain View, CA), and cDNA was generated with oligo-dT and random primers. Real-time quantitative PCR analysis was performed in triplicate per sample (7900HT, ABI, Foster City, CA). The probe and primers for *RRMI* were those previously described (Bepler et al., J Clin Oncol 2004; 22:1 878-1885). Commercially available primers and probes were used for expression analysis of all other target genes (Table 5). The relative amount *of RRMI* and *TS* mRNA in a sample was determined by comparing the threshold cycle with a standard curve as described (Bepler et al., J (Clin Oncol 2004; 22:1878- 1885). For the remaining genes, relative quantification was performed by comparison of the test samples to a single calibrator sample in a fluidic card assay. Negative controls without a cDNA template were included in all experiments.

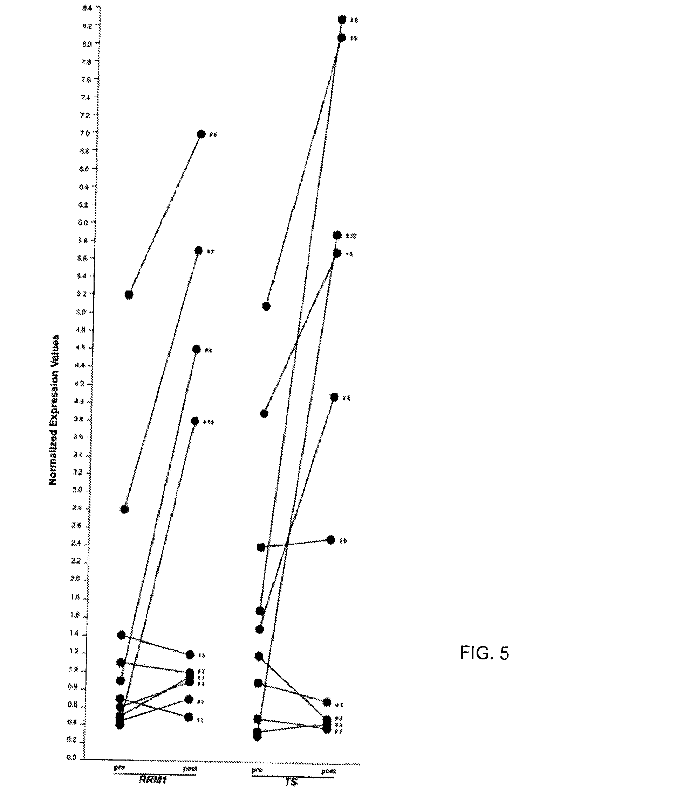

There was a significant correlation between pre- and post-treatment levels for both genes(Spearman's *rho* =0.786, *p* =0,025) suggesting that chemotherapy gene expression levels are representative of pretreatment. levels. However, gene expression levels appear to increase with treatment (Figure 5). All subsequent analyses were performed on the 35 post-treatment specimens. The ranges of expression and other marker characteristics for all 14 genes are summarized in Table 5. The mRNA levels of *RRMI* were significantly correlated with disease response *(rho =* 0.649, *p* = <0.001); i.e., low levels were predictive of tumor size reduction and high levels of tumor growth (Figure 3B). Of the 18 patients with *RRMI* expression equal to or below the median of 1.68, 10had a PR. or CR,while only 2 of the 17with high *RRMI* expression responded. The mRNA levelsof *TS* were likewise correlated with disease response (*rho*=0.454, *p*= 0.006) (Figure 3C); however, after using a Bonferroni adjustment for multplicity of data analysis, the *p-value* was above the level of 0.0036. Of the 18 patients with *TS* expression equal to or below the median of 3.40, 7 had a PR or CR, while only5 of the 17 with high *TS* expression responded.

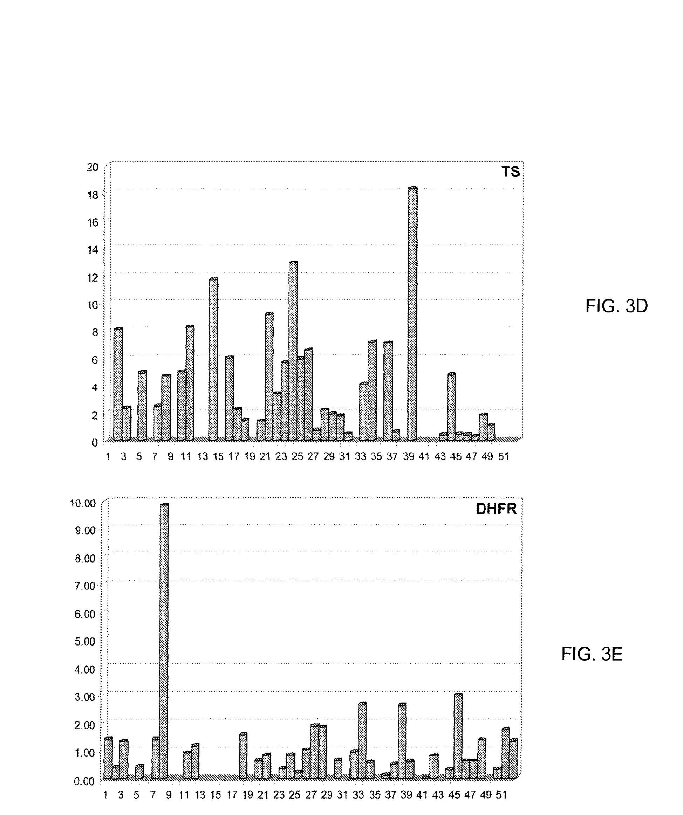

Surprisingly, the expression levels of all other genes were not significantly correlated with disease response (Figures 3D & E), though they are also considered to be targets of the administered therapeutics; the top seven genes listed in Table 5 are in the gemcitabine metabolic pathway, while the bottom seven in Table 5 are involved in pemetrexed metabolism, and only RRMI and TS showed a significant correlation (see the rightmost column of Table 5).

**Table 5: mRNA. Gene Expression Characteristics and Association with Radiographic Disease Response**

| | Gene Name | Probe set ID | N | Min | Max | Median | Mean | Spearman's rho | p-value |
|---|---|---|---|---|---|---|---|---|---|
| Clinical Response | | | 49 | 95% (increase) | -100% (decrease) | -19% | -19% | | |
| RRM1 | regulatory subunit of ribonucleotide reductase | * | 35 | 0.37 | 7.93 | 1.68 | 2.72 | 0.649 | ≤0.001 |
| RRM2a | catalytic subunit of ribonucleotide reductase | Hs00367247-ml | 32 | 0.00 | 5.42 | 0.90 | 1.38 | -0.017 | 0.924 |
| RRM2b | P53-inducible catalytic subunit of ribonucleotide reductase | Hs00153082-ml | 32 | 0.00 | 12.24 | 1.34 | 1.99 | 0.276 | 0.125 |
| DCK | deoxycytidine kinase | Hs00176127-ml | 32 | 0.46 | 40.39 | 3.82 | 5.52 | 0.130 | 0.476 |
| CDA | cytidine deaminase | Hs00156401-ml | 32 | 0.00 | 118.66 | 2.42 | 10.27 | 0.028 | 0.878 |
| ENT1 (SLC29A1) | equilibration sensitive nucleoside transporter 1 | Hs00191940-ml | 32 | 0.00 | 21.42 | 1.30 | 2.18 | 0.162 | 0.374 |
| 5'-NT (NT5CIA) | cytosolic 5'-nucleotidase | Hs00261369-ml | 32 | 0.00 | 170843.81 | 0.00 | 10.89 | -0.081 | 0.657 |
| TS | thymidylate synthase | Hs00426591-ml | 35 | 0.32 | 18.31 | 3.40 | 4.46 | 0.454 | 0.006 |
| DHFR | dihydrofolate reductase | Hs00758822-sl | 32 | 0.00 | 9.83 | 0.85 | 1.29 | 0.166 | 0.362 |
| GARFT | phosphoribosylglycinamide formyl | Hs00531926-ml | 32 | 0.17 | 21.93 | 1.19 | 2.28 | 0.002 | 0.988 |
| FPGS | folylpolyglutamate synthase | Hs00191956-ml | 32 | 0.36 | 32.75 | 2.23 | 3.50 | 0.222 | 0.220 |
| EN12 (SLC29A2) | equilibration sensitive nucleoside transporter 2 | Hs00155426-ml | 32 | 0.11 | 41.30 | 5.16 | 7.76 | 0.125 | 0.494 |
| RFC1 (SLC19A1) | reduced folate carrier | Hs00161870-ml | 32 | 0.01 | 33.47 | 0.12 | 2.23 | 0.096 | 0.600 |
| γ-GH | gamma-glutamyl hydrolase | Hs00608257-ml | 32 | 0.07 | 33.52 | 2.47 | 5.75 | -0.015 | 0.934 |

| | | | | | | | | | |
|---|---|---|---|---|---|---|---|---|---|
| * 5'-FAM-TTTGC TCTTT GGATT CCGGA TCTCT TCA-TAMRA-3' | | | | | | | | | |

### Results

The pre-operative efficacy of a non-platinum doublet, namely gemcitabine and pemetrexed, was investigated in patients with surgically resectable NSCLC. The nationale for this combination was that both agents are antimetabolites with relatively well known mechanisms of action, both are well tolerated, and both are efficacious and already integrated into patient care. Prior randomized phase III neondjuvant studies that utilized platimum-based chemotherapy had reported response rates of 33%,5 41%,7 49%.6 and 64%,4 Approximately 75% of patients received all planned chemotherapy and the chemotherapy-ralated mortality was approximately 2%. These response rates appear higher than the response rate of 35%; however, 18/52 patients in this study had stage III disease, while two of the referenced trials did not include stage III patients,5,7 and the proportion of stage III patients in the other two trials was 7%6 and 47%4 respectively. In two earlier randomized neoadjuvant trials with platinum-based therapy in patients with stage III disease, the reported response rates were 53% (16/30)16 and 35% (9/26) (Roth et al., ,J Natl Cancer Inst 1994; 86:673-680). The expected response rate was set *a priori* at 50%; a goal, which was not achieved. Therefore, the combination of gemcitabine and pemetrexed is unlikely to be superior to platinum-containing doublets if given to an unselected group of patients with resectable NSCLC.

Eighty-seven percent (45/52) of patients in the trial received the planned 4 cycles of therapy. Five patients had less than 4 cycles because of treatment-related grade 3 toxicities, and of these four had surgery with a complete resection. One patient died prior to surgery from a presumed viral pneumonitis. Thus, the combination of gemeitabine and pemetrexed is well tolerated and is deliverable as tour bi-weekly treatments to a proportion of patients that it at least equal to the proportion of patients that can receive a platinum-containing doublet.

These pharmacogenomic studies indicate that the magnitude of tumor response to gemcitabine and pemetrexed is associated with tumoral expression of the genes RRM1 and TS. Patients whose tumors express these genes at low levels are more likely to experience a reduction in tumor size compared to those with high levels of gene expression. This association was significant for the gene RRM1, but failed to reach the significance level of 0.0036 for TS. Since a total of 14 genes were evaluated in these patients, the significance level was adjusted according to Bonferroni. This is a conservative approach to adjustment for Multiplicity of data analysis, and most correlative investigations are conducted under less stringent conditions. Thus the correlation coefficient of (-)0.454 seen for the association between TS expression and response to treatment is remarkable and requires further exploration.

In summary, the clinical correlative investigations described extend the previously known relationship between RRM1 expression and gemcitabine efficacy to the combination of gemcitabine and pemetrexed. They also provide reasonable evidence for an association between TS expression and efficacy of this combination. In an unselected group of patients with resectable NSCLC, gemcitabine and pemetrexed provide an objective radiographic response rate of 35% with good tolerability.

### Additional References

Bepler et al ., J Clin Oncol 2004;22:1878-85.
Bepler et al., J Clin Oncol 2006; 24:4731-4737
Camp et al, Nat Med 2002;8:1323-27.
Ceppi et al., Cancer 2006; 107:1589-96.
Chen et al., N Engl J Med 2007; 356:11-20
Coombes et al., Nat Med 2007; 13:1276-1278
Edler D et al., Clin Cancer Res 2000;6(2):488-92.
Gautam and Bepler, Cancer Res 2006;66:6497-502.
Gautam et al., Oncogene 2003;22:2135-42.
Kato et al., N Engl J Med 2004;350(17):1713-21.
Olaussen et al., N Engl J Med 2006;355:983-91.
Potti et al., N Engl J Med 2006; 355:570-580
Potti et al., Nat Med 2006; 12:1294-1300
Rimm, Nat Biotechnol 2006;24(8):914-6.
Rosell et al., N Engl J Med 1994; 330:153-158
Simon et al., Chest 2005;127(3):978-83,
Zhou et al., Cancer Research 1995:55(6):1328-33.

### OTHER EMBODIMENTS

It is to be understood that while the invention has been described in conjunction with the detailed description trereof,the foregoing description is intended to illustrate and not limit the scope of the invention.

## Claims

1. A method for predicting a subject's response to a treatment comprising administration of gemcitabine and pemetrexed, the method comprising;
a. determining a level of Ribonucleotide Reductase M1 (RRM1) and thymidylate synthase (TS) gene expression in a tumor sample from the subject; and
b. predicting the subject's response to the treatment based on the level of RRM1 and TS gene expression in the tumor sample; wherein
if the level of RRM1 gene expression is less than or equal to a reference level of RRM1 gene expression; and
if the level of TS gene expression is less than or equal to a reference level of TS gene expression;
then the subject is likely to have a positive response to the treatment.

2. The method of claim 1, wherein the subject has an epithelial malignancy.

3. The method of claim 1, wherein the subject has a lung cancer, a breast cancer, a colorectal cancer, a head and neck cancer, or an ovarian cancer.

4. The method of claim 3, wherein the lung cancer is non-small cell lung cancer.

5. The method of claim 1, wherein the reference level of RRM 1 gene expression is a median level of RRM 1 gene expression.

6. The method of claim 1, wherein the reference level of TS gene expression is a median level of TS gene expression.

## Patentansprüche

1. Verfahren zur Vorhersage der Reaktion eines Patienten auf eine Behandlung, welche die Verabreichung von Gemcitabin und Pemetrexed beinhaltet, wobei das Verfahren aufweist:
a. Bestimmen eines Spiegels der Genexpression von Ribonucleid-Reduktase M1 (RRM1) und Thymidylat-Synthase (TS) in einer Tumorprobe von dem Patienten; und
b. Vorhersage der Reaktion des Patienten auf die Behandlung auf der Basis des Genexpressionsspiegels von RRM1 und TS in der Turnorprobe; wobei,
wenn der RRM1-Genexpressionsspiegel kleiner oder gleich einem Bezugswert des RRM1-Genexpressionsspiegels ist; und
wenn der TS-Genexpressionsspiegel kleiner oder gleich einem Bezugswert des TS-Genexpressionsspiegels ist;
der Patient dann wahrscheinlich positiv auf die Behandlung anspricht.

2. Verfahren nach Anspruch 1, wobei der Patient an einer malignen Epithelerkrankung leidet.

3. Verfahren nach Anspruch 1, wobei der Patient an Lungenkrebs, Brustkrebs, Kolorektalkrebs, Kopf- und Halskrebs oder Eierstockkrebs leidet.

4. Verfahren nach Anspruch 3, wobei der Lungenkrebs nichtkleinzelliges Lungenkarzinom ist.

5. Verfahren nach Anspruch 1, wobei der Bezugswert des RRM1-Genexpressionsspiegels ein Medianwert der RRM1-Genexpression ist.

6. Verfahren nach Anspruch 1, wobei der Bezugswert des TS-Genexpressionsspiegels ein Medianwert der TS-Genexpression ist.

## Revendications

1. Procédé pour prévoir une réponse d'un sujet à un traitement comprenant l'administration de gemcitabine et de pémétrexed, le procédé comprenant:
a. la détermination d'un niveau d'expression de gène de ribonucléotide réductase M1 (RRM1) et de thymidylate synthase (TS) dans un échantillon de tumeur provenant du sujet; et
b. la prévision de la réponse du sujet au traitement sur la base du niveau d'expression de gène de RRM1 et de TS dans l'échantillon de tumeur; dans lequel:
si le niveau d'expression de gène de RRM1 est inférieur ou égal à un niveau de référence d'expression de gène de RRM1; et
si le niveau d'expression de gène de TS est inférieur ou égal à un niveau de référence d'expression de gène de TS;
alors le sujet est susceptible d'avoir une réponse positive au traitement.

2. Procédé selon la revendication 1, dans lequel le sujet présente une malignité épithéliale.

3. Procédé selon la revendication 1, dans lequel le sujet présente un cancer du poumon, un cancer du sein, un cancer colorectal, un cancer de la tête et du cou ou un cancer de l'ovaire.

4. Procédé selon la revendication 3, dans lequel le cancer du poumon est un cancer du poumon non à petites cellules.

5. Procédé selon la revendication 1, dans lequel le niveau de référence d'expression de gène de RRM1 est un niveau médian d'expression de gène de RRM1.

6. Procédé selon la revendication 1, dans lequel le niveau de référence d'expression de gène de TS est un niveau médian d'expression de gène de TS.
